(19) Europäisches Patentamt
European Patent Office
Office européen des brevets

(11) EP 0 819 703 B1

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(51) Int Cl.$^7$: **C08B 37/00**, A61K 31/716

(21) Anmeldenummer: **97112307.0**

(22) Anmeldetag: **17.07.1997**

(54) **Polymere Glucanäther-Derivate, ihre Herstellung sowie ihre Verwendung**

Ether derivatives of polymeric glucans, their preparation as well as their use

Dérivés éthérifiés de polymères de glucane, procédé de préparation et leur utilisation

(84) Benannte Vertragsstaaten:
**CH DE DK FI FR GB LI SE**

(30) Priorität: **19.07.1996 DE 19629117**

(43) Veröffentlichungstag der Anmeldung:
**21.01.1998 Patentblatt 1998/04**

(60) Teilanmeldung:
**01128699.4 / 1 197 216**

(73) Patentinhaber: **Mibelle AG Cosmetics**
**5033 Buchs (CH)**

(72) Erfinder:
- **Zülli, Fred, Dr.sc. nat.**
  **5024 Küttingen (CH)**
- **Suter, Franz**
  **5312 Döttingen (CH)**

(74) Vertreter: **Lau-Loskill, Philipp, Dipl.-Phys.**
**Berger Dorfstrasse 35**
**41189 Mönchengladbach (DE)**

(56) Entgegenhaltungen:

| | |
|---|---|
| **EP-A- 0 397 880** | **DE-A- 2 152 059** |
| **DE-A- 4 431 251** | **US-A- 4 454 315** |

- **PATENT ABSTRACTS OF JAPAN vol. 015, no. 474 (C-0890), 3. Dezember 1991 (1991-12-03) & JP 03 204804 A (MITSUI TOATSU CHEM. INC.)**
- **S. DEMLEITNER ET AL.: "Synthesis and antitumour activity of sulfoalkyl derivatives of curdlan and lichenan" CARBOHYDRATE RESEARCH, Bd. 226, 30. März 1992 (1992-03-30), Seite 247-252 XP000258120**
- **F. ZÜLLI ET AL.: "Photoprotective effects of CM-glucan on cultured human skin cells" EURO COSMETICS, Bd. 1995, Nr. 11, 1995, Seite 46-50 XP002090000**
- **F. ZÜLLI ET AL.: "CM-Glucan a new yeast polysaccharide for cosmetic use" COSMETICS AND TOILETRIES MANUFACTURE WORLDWIDE,1994, Seite 131,133,134,136 XP002090001**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von polymeren Glucanäther-Derivaten.

Glucan ist eine 1,3-beta-Polyglukose und wird durch einen alkalischen sowie sauren Aufschluß aus Hefezellen gewonnen, wie dieser beispielsweise in der US 5,397,773 A, der US 5,250,436 A oder der US 5,223,491 A beschrieben ist.

Der Hauptnachteil des so isolierten Glucans ist darin zu sehen, daß das Glucan eine extrem geringe Löslichkeit in wäßrigen Systemen besitzt, so daß es dementsprechend beispielsweise für wäßrige formulierte pharmazeutische und/oder kosmetische Zubereitungen nur bedingt geeignet ist.

Um Glucanäther-Derivate herzustellen, schlägt die US 4,454,315 B vor, daß

a) Glucan in Isopropanol unter Zusatz einer alkalischen Lösung behandelt wird,
b) die so erstellte Lösung hiernach während insgesamt 150 Minuten gerührt wird,
c) danach Monochloressigsäure zugesetzt und das so erstellte Reaktionsgemisch bei 60 bis 70 °C für 5 Stunden weiter gerührt wird,
d) das hierdurch erstellte Carboxymethylglucan filtriert und mit einer Mischung aus Methanol und Essigsäure, anschließend mit einer 80 %igen wäßrigen Methanol-Lösung und danach mit einer Mischung aus Methanol und Aceton gewaschen wird und
e) das gewaschene Carboxymethyl-Glucan schonend getrocknet wird.

Das in vorstehender Weise gewonnene Carboxymethyl-Glucan kann nach einer Veröffentlichung in "Cosmetics and Toiletries Manufacture Worldwide, 1994, S. 131, 133, 134 und 136" vielfältig eingesetzt werden. So beschreibt diese Veröffentlichung primär dermatologische Untersuchungen einer 2 %igen wäßrigen Carboxymethylglucan-Lösung und kommt zu dem Ergebnis, daß diese 2 %ige wäßrige Lösung von Carboxymethylglucan dermatologisch unbedenklich ist, so daß dementsprechend in dieser Veröffentlichung vorgeschlagen wird, Carboxymethylglucan in Sonnenschutzmitteln, in Anti-Akne-Mitteln oder in After-Sun-Produkten zu verwenden. Weiterhin deutet diese Veröffentlichung an, daß auch pathologischen Veränderungen, die beim Altern der Haut auftreten, durch Carboxymethyl-Glucan entgegengewirkt werden kann.

Der vorliegenden Erfindung liegt die Aufgabe zugrunde, ein Verfahren zur Herstellung von Glucanäther-Derivaten zur Verfügung zu stellen, mit dem diese Glucanäther-Derivate besonders einfach und mit einem hohen Reinheitsgrad herstellbar sind.

Diese Aufgabe wird erfindungsgemäß durch ein Verfahren mit den kennzeichnenden Merkmalen des Patentanspruchs 1 gelöst.

Das erfindungsgemäße Verfahren zur Herstellung von polymeren Glucanäther-Derivaten, die neben Monomereneinheiten der Formel I auch solche Monomereneinheiten der allgemeinen Formel II aufweisen,

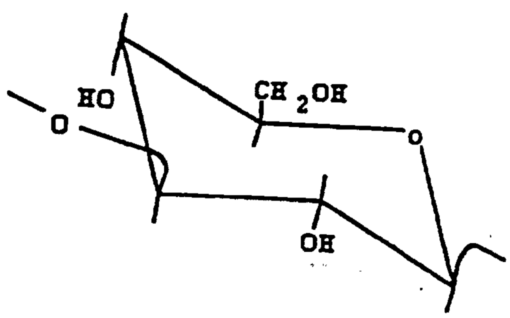

(Formel I)

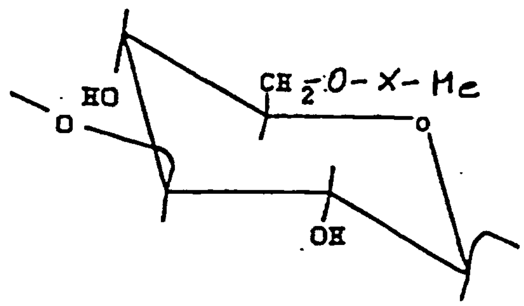

(Formel II)

wobei die Monomereneinheiten der Formel I mit den Monomereneinheiten der Formel II über eine 1,3-beta-glykosidische Bindung verknüpft sind und wobei in Formel II
X eine $-CH_2-COO$-Gruppe, eine $-CH_2-CH_2-COO$-Gruppe, eine

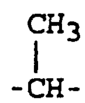

COO-Gruppe und/oder eine $-CH_2-CH_2-SO_3$-Gruppe und Me Wasserstoff, ein Alkali- und/oder ein Erdalkalimetall bedeuten, sieht vor, daß

a) gereinigtes und weitestgehend lipidfreies Glucan in einem $C_1$-$C_4$- Alkohol unter Zusatz einer alkalischen Lösung behandelt wird,
b) hiernach ein Teil der alkalischen Lösung entfernt wird,
c) die dabei anfallende Suspension auf eine Temperatur zwischen 40 °C und 60 °C erwärmt und anschließend bei dieser Temperatur mit einer wäßrigen Lösung eines Salzes einer Halogencarbonsäure oder einer Halogensulfonsäure der nachfolgenden Formeln III

$Y - CH_2-COO\ Me$,

$Y - CH_2-CH_2-COO\ Me$,

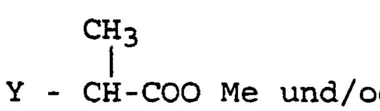 und/oder

$Y - CH_2-CH_2-SO_3\ Me$

(Formeln III)

umgesetzt wird, wobei in den Formeln III Y ein Halogen und Me ein Alkali- und/oder Erdalkalimetall bedeuten,
d) nach einer Reaktionszeit von einer Stunde bis vier Stunden das so gebildete Glucanäther-Derivat gewaschen und daß
e) das gewaschene Glucanäther-Derivat schonend getrocknet wird.

Das erfindungsgemäße Verfahren weist zunächst den entscheidenden Vorteil auf, daß es relativ einfach und mit geringem apparativem Aufwand durchführbar ist. Desweiteren konnte festgestellt werden, daß sich nach dem zuvor beschriebenen erfindungsgemäßen Verfahren die in den Formeln I und II wiedergegebenen polymeren Glucanäther-Derivate besonders schonend und mit hoher Reinheit herstellen lassen, so daß dementsprechend diese Glucanäther-Derivate schnell verfügbar sind. Auch treten bei dem erfindungsgemäßen Verfahren keine unerwünschten Nebenreaktionen, so z.B. eine unerwünschte Spaltung der 1,3-beta-glykosidischen Bindung auf, was dazu führt, daß die polymeren Glucanäther-Derivate besonders reproduzierbar herstellbar sind und keine unerwünschten Nebenprodukte beinhalten. Dementsprechend sind die Glucanäther-Derivate hervorragend für pharmazeutische und/oder kosmetische Zubereitungen einsetzbar, wie dies nachfolgend noch näher beschrieben ist.

Grundsätzlich kann bei dem erfindungsgemäßen Verfahren die vorstehend in der Stufe a) wiedergegebene Behandlung des gereinigten und weitestgehend lipidfreien Glucans unter Zusatz einer alkalischen Lösung bei jeder beliebigen Temperatur durchgeführt werden, wobei es jedoch besonders vorteilhaft ist, wenn diese Behandlung des gereinigten und weitestgehend lipidfreien Glucans bei Raumtemperatur, d.h. in einem Temperaturbereich zwischen etwa 16 °C und etwa 22 °C, durchgeführt wird. Dies hat den entsprechenden Vorteil, daß unerwünschte Nebenreaktionen schon in der ersten Reaktionsstufe a) des erfindungsgemäßen Herstellungsverfahrens vermieden werden, so zum Beispiel eine unerwünschte Hydrolyse der 1,3-beta-glykosidischen Bindung, so daß dementsprechend die Reproduzierbarkeit und die Ausbeute des erfindungsgemäßen Verfahrens noch weiter verbessert wird.

Wird bei dem erfindungsgemäßen Verfahren der vorstehend unter Punkt a) wiedergegebene Reaktionsschritt bei Raumtemperatur ausgeführt, so variiert man abhängig von der zu erzielenden Ausbeute des jeweils zu synthetisierenden polymeren Glucanäther-Derivates und dessen Reinheit die Reaktionszeit zwischen etwa 10 Stunden und 25 Stunden, vorzugsweise zwischen etwa 12 Stunden und 20 Stunden.

Vorstehend ist bei dem erfindungsgemäßen Verfahren bei dem zuvor erwähnten ersten Reaktionsschritt a) beschrieben, daß das gereinigte und weitestgehend lipidfreie Glucan in einem $C_1$-$C_4$-Alkohol suspendiert wird. Hierfür wird vorzugsweise Isopropanol angewendet, wobei das Massenverhältnis von Glucan zu dem $C_1$-$C_4$-Alkohol bzw. zu dem Isopropanol zwischen 1:10 bis 1:30, vorzugsweise zwischen 1:15 bis 1:25, variiert. Ein derartiges konkretes Massenverhältnis bewirkt unter anderem, daß bei dieser Ausführungsvariante des erfindungsgemäßen Verfahrens eine quantitative Umsetzung der Glucan-Suspension mit der vorzugsweise als wäßrige Natronlauge vorliegenden alkalischen Lösung erfolgt, was wiederum einen Einfluß auf die Reinheit des so hergestellten Glucanäther-Derivates sowie auf die Wirtschaftlichkeit des erfindungsgemäßen Verfahrens hat.

Um bei dem erfindungsgemäßen Verfahren im eingangs wiedergegebenen Reaktionsschritt a) eine unerwünschte Nebenreaktion, so zum Beispiel die Spaltung der im Glucan enthaltenen 1,3-beta-glykosidischen Bindung, zu unterdrücken, sieht eine Weiterbildung des erfindungsgemäßen Verfahrens vor, daß außer und/oder zusätzlich zu dem zuvor erwähnten Temperaturbereich sowie den zuvor beschriebenen Reaktionszeiten als alkalische Lösung eine 20 Gew.%ige bis 35 Gew.%ige wäßrige Natronlauge in einem Massenverhältnis von Glucan zu Natronlauge zwischen 1:1,5 und 1:3 verwendet wird.

Bei einer anderen Weiterbildung des erfindungsgemäßen Verfahrens wird, wie vorstehend bei dem erfindungsgemäßen Verfahren im Reaktionsschritt b) beschrieben, nach dem Entfernen des Teiles der alkalischen Lösung und vor dem Erwärmen der Suspension eine weitere Menge eines $C_1$-$C_4$-Alkohols und insbesondere des Isopropanols dem Reaktionsgemisch zugesetzt, so daß anschließend dann die um die entsprechende Alkoholmenge ergänzte Suspension auf eine Temperatur zwischen 40 °C und 60 °C erwärmt wird, wie dies bereits vorstehend beim erfindungsgemäßen Verfahren unter dem Reaktionsschritt c) beschrieben ist. Hierbei dient diese Maßnahme ebenfalls dazu, einerseits die Hydrolyse zu unterdrücken und andererseits eine gezielte Reaktion in der Stellung 6 herbeizuführen, so daß dementsprechend durch Anwendung dieser Verfahrensvariante hochreine Glucanäther-Derivate hergestellt werden können.

Grundsätzlich besteht bei der zuvor beschriebenen Weiterbildung des erfindungsgemäßen Verfahrens die Möglichkeit, daß die zugesetzte Menge des $C_1$-$C_4$ Alkohols nicht der Menge der teilweise entfernten alkalischen Lösung entspricht, wobei jedoch festgestellt werden konnte, daß besonders gute Ausbeuten dann bei dem erfindungsgemäßen Verfahren erzielbar sind, wenn die zugesetzte Menge des $C_1$-$C_4$- Alkohols der Menge der teilweise entfernten alkalischen Lösung entspricht.

Bei dem erfindungsgemäßen Verfahren wird vorstehend unter Punkt d) vorgesehen, daß das nach dem erfindungsgemäßen Verfahren hergestellte Glucanäther-Derivat nach Ablauf einer Reaktionszeit zwischen 1 Stunde und 4 Stunden gewaschen wird. Grundsätzlich kann zum Waschen des nach dem erfindungsgemäßen Verfahren hergestellten Glucanäther-Derivates jedes geeignete organische Lösungsmittel verwendet werden, in dem das gebildete Glucanäther-Derivat nicht oder nur bedingt löslich ist.

Eine besonders geeignete Weiterbildung des erfindungsgemäßen Verfahrens schlägt vor, daß zum Waschen des Glucanäther-Derivates der Zusatz einer Alkohol-/Wasser-Mischung erfolgt, wobei insbesondere Isopropanol-/Wasser-Mischungen in einem Volumenverhältnis von Isopropanol zu Wasser von 1:1 bis 1,5:1 besonders bevorzugt sind.

Um bei dem erfindungsgemäßen Verfahren nach Abschluß der vorstehend unter Punkt d) angegebenen Reaktionszeit ein Abbruch der Reaktion sicher und reproduzierbar herbeizuführen, sieht eine andere Weiterbildung des erfindungsgemäßen Verfahrens vor, daß das Reaktionsgemisch nach Ablauf der Reaktionszeit von 1 Stunde bis zu 4 Stunden durch Zusatz einer Säure, vorzugsweise durch Zusatz von konzentrierter Salzsäure, auf einen pH-Wert zwischen 6,8 und 7,5 neutralisiert wird.

Um bei dem erfindungsgemäßen Verfahren das hiernach hergestellte gewaschene und/oder neutralisierte Glucanäther-Derivat aus dem Reaktionsgemisch zu isolieren, wird bevorzugt das so hergestellte und im wäßrigen System lösliche Glucanäther-Derivat durch Zusatz eines $C_1$-$C_4$- Alkohols, vorzugsweise durch Zusatz von Isopropanol,

aus der wäßrigen Reaktionsmischung ausgefällt. Durch diese Variante des erfindungsgemäßen Verfahrens kann das so hergestellte Glucanäther-Derivat nahezu quantitativ aus dem Reaktionsgemisch entfernt werden, wobei ein derartig isoliertes Glucanäther-Derivat dann noch eine hohe Reinheit aufweist.

Bezüglich der sich an die Isolierung anschließende Trocknung ist festzuhalten, daß diese Trocknung des nach dem erfindungsgemäßen Verfahrens hergestellten Glucanäther-Derivats vorzugsweise schonend zu erfolgen hat. Hier hat sich gezeigt, daß eine Vakuumtrocknung in einem Temperaturbereich zwischen 40 °C und 80 °C ganz besonders diesen Anforderungen genügt, so daß eine derartige Trocknung bei dem erfindungsgemäßen Verfahren bevorzugt wird.

Insbesondere weist das nach dem erfindungsgemäßen Verfahren hergestellte Glucanäther-Derivat, einen Durchschnittssubstitutionsgrad von 0,75 ± 0,1, auf. Die allgemeine Formel V gibt ein bevorzugtes Glucanäther-Derivat wieder, dessen Molekulargewicht zwischen 20.000 und 2.000.000, vorzugsweise zwischen 50.000 und 500.000 variiert. In der Formel V haben X und Me die selbe Bedeutung, wie diese vorstehend bereits angegeben ist.

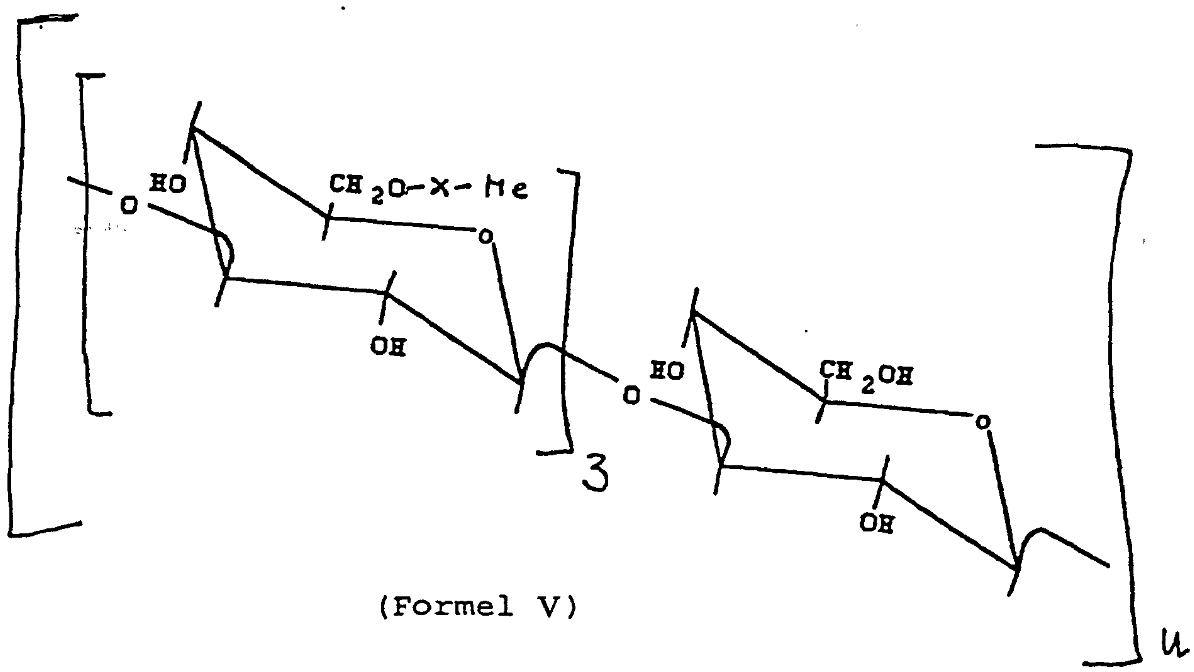

(Formel V)

Das bei dem erfindungsgemäßen Verfahren als Ausgangssubstanz eingesetzte gereinigte und weitestgehend lipidfreie Glucan kann nach den an sich bekannten Verfahren hergestellt werden.

Die nach dem erfindungsgemäßen Verfahren hergestellen Glucanäther-Derivate, insbesondere das Carboxymethylglucan, enthalten entsprechenden Monomereneinheiten, die über eine 1,3-beta-glykosidische Bindung miteinander verknüpft sind. Diese Formulierung soll jedoch auch solche Derivate umfassen, bei denen die Monomereneinheiten linear über eine 1,3-beta-glykosidische Bindung und zusätzlich noch verzweigt über eine 1,6-beta-glykosidische Bindung verknüpft sind, wobei jedoch der Anteil der verzweigten 1,6-beta-glykosidischen Bindungen wesentlich geringer ist als der Anteil der linearen 1,3-beta-glykosidischen Bindungen und insbesondere zwischen 0 % und 20 %, vorzugsweise zwischen 4 % und 10 %, bezogen auf den Anteil der 1,3-glykosidischen Bindungen, ausmacht.

Die Erfindung wird nachfolgend anhand eines Ausführungsbeispieles näher erläutert.

**Herstellung von Carboxymethyl-Glucan**

1 kg Glucan wurde in 20 l Isopropanol unter Rühren in der Kälte suspendiert und mit 2 kg 30 Gew.%iger wäßriger Natriumhydroxidlösung während 16 Stunden bei Raumtemperatur (16 °C - 22 °C) behandelt.

Nach Abschluß dieser alkalischen Behandlung wurde nach einer kurzen Verweilzeit von etwa 20 Minuten der über der Suspension anstehende Überstand von 8 l des wäßrigen alkalischen Isopropanols abdekantiert. Hiernach erfolgte die Zugabe von 8 l Isopropanol.

Unter Rühren wurde dann die Suspension auf 60 °C erwärmt.

Nach Erreichen der Endtemperatur wurden 1,5 kg des Natriumsalzes der Chloressigsäure, gelöst in 1,5 kg Wasser, zugesetzt.

Nach einer Gesamtreaktionszeit von 2,5 Stunden bei 60 °C wurde das Reaktionsprodukt sedimentiert und

die darüber befindliche Flüssigkeit abdekantiert. Anschließend erfolgte ein Zusatz von 4 l einer wäßrigen Isopropanollösung (V:V, 50 % : 50 %).

Nachdem das Reaktionsprodukt gewaschen war, wurde das Natriumsalz des Carboxymethyl-Glucans in 20 l Wasser gelöst. Es erfolgte ein Zusatz zu der Lösung einer 10 N Salzsäure, derart, daß der pH-Wert der Lösung auf einen Wert von 7 eingestellt wurde.

Aus der neutralisierten, wäßrigen Lösung wurde das Natriumsalz des Carboxymethyl-Glucans durch Zusatz von 40 l Isopropanol ausgefällt.

Nach Abtrennung des ausgefällten Natriumsalzes des Carboxymethyl-Glucans wurde dieses im Vakuum-Trockenschrank bei 60 °C getrocknet.

Die Ausbeute an Natriumsalz des Carboxymethyl-Glucans betrug 1,3 kg.

Das Natriumsalz des Carboxymethyl-Glucans wurde analysiert. Hierbei zeigte sich, daß das Produkt einen Durchschnittssubstitutionsgrad von 0,75 ± 0,1 aufwies.

**In-Vivo-Test von 3 Formulierungen in bezug auf Ihre Wirksamkeit gegen den UVA-induzierten oxidativen Streß**

Es wurden drei unterschiedliche Creme formuliert, wobei die Creme folgende Zusammensetzungen aufwiesen:

| | Creme A |
|---|---|
| 1. Komponente | 0,5 % Phenoxyethanol<br>0,3 % Imidazolidinyl Urea<br>0,02 % Triethanolamine<br>2 % Alcohol<br>1 % Propylene Glycol<br>87,46 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogenated Polyisobutene |
| 3. Komponente | 0,02 % Parfum |

| | Creme B |
|---|---|
| 1. Komponente | 0,5 % Phenoxyethanol<br>0,3 % Imidazolidinyl Urea<br>0,02 % Triethanolamine<br>2 % Alcohol<br>1 % Propylene Glycol<br>0,2 % Sodium Carboxymethyl Betaglucan<br>87, 46 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogenated Polyisobutene |
| 3. Komponente | 0,02 % Parfum |

(fortgesetzt)

| | Creme A |
|---|---|
| | Creme C |
| 1. Komponente | 0,5 % Phenoxyethanol<br>0,3 % Imidazolidinyl Urea<br>0,02 % Triethanolamine<br>2 % Alcohol<br>1 % Propylene Glycol<br>0,04 % Sodium Carboxymethyl Betaglucan<br>87,62 % Aqua |
| 2. Komponente | 4 % PEG-5 Glyceryl Stearate<br>1 % Cetyl Alcohol<br>1 % Stearic Acid<br>2 % Paraffinum Liquidum<br>0,5 % Hydrogenated Polyisobutene |
| 3. Komponente | 0,02 % Parfum |

Die vorstehend wiedergegebenen %-Angaben beziehen sich alle auf Gewichtsprozent und die dort verwendeten Produktbezeichnungen entsprechen der INCI-Declaration, wie sie dem "International Cosmetic Ingredient Dictionary", 1995, veröffentlicht von "The Cosmetic, Toiletry, and Fragrance Association", Washington, zu entnehmen sind.

Zur Herstellung der Cremen A bis C wurde einheitlich wie folgt verfahren:

Die erste Komponente wurde jeweils in einen Reaktor eingefüllt und auf ca. 60 °C erwärmt. Unter Rühren wurden die auf etwa 70 °C erwärmte zweite Komponente zugegeben. Die vereinigten Komponenten wurden sodann in dem Reaktor homogenisiert. Anschließend erfolgte unter Rühren ein Abkühlen des Gemisches auf ca. 35 °C. Nach Erreichen dieser Temperatur wurde die dritte Komponente zugegeben und unter Rühren auf 25 °C abgekühlt.

Auf definierten Hautarealen (Durchmesser ca. 2,5 cm) der Unterarminnenseiten von 10 Probanden wurden für 5 Tage zweimal täglich ca. 2 $mg/cm^2$ die jeweilige Creme A bis Creme C appliziert. Zwei Felder blieben unbehandelt und dienten als Kontrolle.

Am fünften Tag erfolgte 30 Minuten nach der letzten Applikation der Creme A bis C eine Bestrahlung mit UVA-Licht (10 $J/cm^2$; Strahlungsquelle: Multiport, Modell 601, Solar Light, USA). Ein Testfeld wurde jeweils nicht bestrahlt.

Nach Extraktion des Squalens und des Squalenhydroperoxids mit 1 ml Ethanol wurden hochdruckflüssigkeitschromatographisch die Konzentrationen des Squalens und des Squalenhydroperoxids in den Extrakten der unterschiedlich behandelten definierten Hautarealen bestimmt.

Die Squalen-Bestimmung erfolgte nach H.P. Nissen et al, Chromatographia 11/12; 686-690; 1990, wobei für die Bestimmung folgende HPLC-Bedingungen eingehalten wurden:

| | |
|---|---|
| Flußrate | 1 ml/min |
| Säule | LiChrospher RP 18 (5 µm); 4,6 x 125 mm |
| Eluent | Acetonitril/i-Propanol (50/50) |
| Detektion | 210 nm |

Die Bestimmung des Squalenhydroperoxids erfolgte nach J.R. Zhang et al, Free Radic. Biol. Med. 1: 1-10; 1995, A.E. Holley et al, Free Rad. Res. Comms. Vol. 15., No. 1., 51-63; 1991 und C. Colin et al, IFSCC-Konferenz 1994, Venedig, A 105, wobei eine Nachsäulenderivatisierung mit Isoluminol/Mikroperoxidase durchgeführt wurde, wobei die Detektion durch Chemilumineszenz erfolgte. Hierbei galten folgende Bedingungen:

| | |
|---|---|
| Flußrate | 1 ml/min |
| Säule | LiChrospher RP-Select B (5 µm); 4,6 x 125 mm |
| Eluent | Methanol |

(fortgesetzt)

| | |
|---|---|
| Derivatisierungs reagenz | Isoluminol (1 mM) und Mikroperoxidase (10 µg/ml) gelöst in 100 mM Borat-Puffer (pH 10)/Methanol (70/30) |
| Flußrate/ Derivatisierungsreagenz | 0,8 ml |
| Detektion | Chemilumineszenz |

Als Ergebnis der Untersuchung ist festzuhalten, daß in den Extrakten, die von den mit den Cremen B und C behandelten Hautarealen gewonnen wurden, nur geringe Mengen an Squalenhydroperoxids nachgewiesen werden konnten. Hingegen ließen sich in solchen Extrakten, die aus der mit der Creme A behandelten Hautareal sowie aus den unbehandelten Hautarealen nach Bestrahlung gewonnen wurden, größere Mengen an Squalenhydroperoxid analysieren.

Da Squalenhydroperoxid durch reaktive Sauerstoffspezies gebildet wird, kann aus diesen Ergebnissen geschlossen werden, daß die Creme B und C, die den Wirkstoff Natriumsalz des Carboxymethyl-Glucans enthalten, in der Lage sind, die Bildung von Squalenhydroperoxiden durch freie Radikale deutlich zu reduzieren.

In der folgenden Tabelle 1 sind die Ergebnisse der Untersuchung als prozentuale Verhinderung der Peroxidation der mit den Cremen B und C behandelten Arealen relativ zu dem mit der Creme A behandelten Areal ausgedrückt.

Tabelle 1

| Produkt | Verhinderung der Peroxidation (in %) bezogen auf die mit Creme A behandelten Hautarealen |
|---|---|
| Creme B | 94,9 |
| Creme C | 58,9 |

Die vorstehend in der Tabelle 1 wiedergegebene Verhinderung der Peroxidation wird wie folgt berechnet:

100 x (SQOOH (Creme A) - SQOOH (Creme B oder Creme C))/SQOOH

(Creme A)

SQOOH = Squalenhydroperoxidkonzentration/Squalenkonzentration

Weder bei der Creme B noch bei der Creme C konnte während der Anwendung bei den 10 Probanden, deren Alter zwischen 18 Jahren und 40 Jahren betrug, irgendeine Hautunverträglichkeit oder eine Hautreizung beobachtet werden.

**In-Vivo-Test von zwei Formulierungen in bezug auf ihre Wirksamkeit als Anti-Aging-Mittel**

Zum Nachweis der Wirksamkeit von Glucanäther-Derivate enthaltenden Zusammensetzungen wurden zwei cremeartigen Formulierungen untersucht, wobei die nachfolgend wiedergegebene cremeartige Zusammensetzung mit der Bezeichnung 2LA als Wirkstoff das Natriumsalz des Carboxymethylbetaglucans, wie dieses vorstehend durch die Formel VI charakterisiert ist, enthält, während die ansonsten identisch aufgebaute cremeartige Zusammensetzung mit der Bezeichnung 2LB diesen zuvor genannten Wirkstoff nicht aufwies.

Die beiden untersuchten Zusammensetzungen 2LB und 2LA, die analog zum vorstehenden Beispiel hergestellt wurden, wiesen die nachfolgend aufgelisteten Inhaltsstoffe auf, wobei die Produktbezeichnungen der INCI-Declaration, wie sie dem zuvor genannten Dictionary zu entnehmen ist, entsprechen.

| INCI-Namen | 2LB Gew.% | 2LA Gew.% |
|---|---|---|
| Aqua | 87,5 | 87,46 |
| Alcohol | 2 | 2 |
| Propylene Glycol | 1 | 1 |
| Sodium Carboxymethyl Betaglucan | 0 | 0,04 |
| Phenoxyethanol | 0,5 | 0,5 |

(fortgesetzt)

| INCI-Namen | 2LB Gew.% | 2LA Gew.% |
|---|---|---|
| Imidazolidinyl Urea | 0,3 | 0,3 |
| PEG-5 Glyceryl Stearate | 4 | 4 |
| Cetylalcohol | 1 | 1 |
| Stearic Acid/Palmitic Acid | 1 | 1 |
| Paraffin | 2 | 2 |
| hydriertes Polyisobuten | 0,5 | 0,5 |
| Parfum | 0,2 | 0,2 |

Zehn gesunde weibliche Testpersonen in einem Alter zwischen 61 und 75 Jahren trugen zweimal täglich über einen Zeitraum von 28 Tagen auf ausgewählte Testgebiete auf den Unterarminnenseiten und im Gesicht (Augenfalten) die zuvor beschriebenen Zusammensetzungen 2LA und 2LB auf.

Zu Beginn der Studie wurde vor der ersten Applikation der Zusammensetzungen die Hautelastizität, die Faltentiefe sowie die Hautrauhigkeit gemessen, wobei diese zuvor genannten drei Parameter nach 14 Tagen und 28 Tagen nach Applikation wiederholt wurden.

Die Testpersonen wurden angewiesen, drei Tage vor Beginn der Prüfung und während der gesamten Testzeit (28 Tage) keine anderen Externa auf die Testgebiete aufzutragen. Zur Reinigung durfte in den Testgebieten nur Wasser verwendet werden.

Die letzte Messung nach 28 Tagen erfolgte 8 Stunden nach der letzten Applikation des jeweiligen Produktes.

Die Testareale der Unterarminnenseiten wurden zweimal pro Woche mit einem Sonnensimulator mit 0,75 MED (minimale Erythemdosis) bestrahlt. Die zuvor angesprochenen Parameter wurden wie folgt gemessen:

a) Messung der Hautelastizität (Hautstraffung)

Die Elastizität der Haut wurde mit dem Cutometer SEM 474 (Hersteller: Courage & Khazaka Electonic GmbH, Köln) gemessen. Hierbei wurde die Hautoberfläche durch ein angelegtes Vakuum in die Öffnung (2 mm, innerer Durchmesser) einer speziellen Sonde gezogen. Die Eindringtiefe der Haut in die Sonde wurde ohne mechanische Einwirkung reibungslos optisch erfaßt. Die Messungen wurden mit konstanten Unterdruck (500 mbar) bei einer Meßzeit von 1 Sekunde durchgeführt. Anschließend wurde der Unterdruck für 1 Sekunde abgeschaltet und der Meßzyklus dann noch fünfzigmal wiederholt.

Zur Auswertung wurden die 50 maximalen und 50 minimalen Amplituden verwendet. Die Datensätze (für jeden Probanden und jeden Meßtag) wurden dann an eine logarithmische Funktion des Typs $y = 1/a\ (\ln x + b)$ angepaßt, wobei x = maximale Amplitude bzw. minimale Amplitude und y = Anzahl der Meßzyklen entspricht. Die Anpassung geschah durch die Minimierung der Summe der Restwerte für die angepaßte Kurve (Programm:Statgraphics Plus for Windows-Version 3.0 - Manugistics, USA).

Bei allen Anpassungen war der Betrag des Korrelationskoeffizienten größer als 0,9. Die Werte von a und b der individuellen logarithmischen Funktionen wurden dann für jedes Testareal und jeden Meßzeitpunkt gemittelt. Aus den Mittelwerten wurden die logarithmischen Funktionen gezeichnet und miteinander verglichen. Die Flächen unter den Funktionen wurden durch Bildung des Integrals zwischen 0 und 100 nach

$$y = \int (\ln x + b)/a\ dx = (bx + x\ln x)/a$$

sowohl für die Maxima als auch für die Minima berechnet (Progamm:Mathcad 6.0 Mathsoft, USA).

b) Messung der Faltentiefe

Die Bestimmung der Faltentiefe erfolgt mit dem Hautoberflächenmeßgerät OFR 01 (Romano GmbH, Köln). Die Bestimmung der Faltentiefe erfolgte mit Hilfe der Profilometrie. Zur Beschreibung der Faltentiefe wird der Parameter Rt (DIN 4768/1) gewählt. Rt ist die maximale Rauhtiefe, d.h. der Abstand zwischen der Linie der Erhebung und der Linie der Vertiefung innerhalb der Meßstrecke. Da eine direkte Messung an den Hautoberflächen nicht möglich ist, wurden entsprechende Hautabdrücke mit Silasoft N (Hersteller: Detax-Dentol, Karlsruhe) angefertigt und unter den nachfolgenden Bedingungen vermessen:

| | |
|---|---|
| Meßweg | 15.000 µm |
| auswertbarer Weg | 12.500 µm |
| Schrittweite | 2,5 µm |
| Gesamtschritte | 5.000 |
| Zahl der Messungen pro Abdruck | 11 |

c) Messung der Hautrauhigkeit

Die Bestimmung der Hautrauhigkeit erfolgte mit dem Skin Visiometer VS 400 (Courage & Khazaka Electronic GmbH, Köln).

Das Meßprinzip beruht auf der Durchleuchtung eines sehr dünnen, speziell angefärbten Silikonhautabdrukkes. Das Licht wird dabei von dem Silikonmaterial, je nach dessen Stärke, entsprechend absorbiert. Der Silikonhautabdruck bildet die Höhen und Tiefen der Haut als Negative ab, d.h. Hautfältchen sind im Abdruck höher, dementsprechend ist das Silikonmaterial an dieser Stelle dicker. Die Meßmethode basiert auf dem Bouguer-Lambertschen Absorptionsgesetz.

Durch Sichtbarmachung der Lichtabsorption, z.B. durch eine schwarz/weiß CCD-Kamera auf einem PC-Monitor, ist es möglich, die Höhe und Tiefen jedes Punktes des Abdruckes durch eine entsprechende Einstufung in eine Grauwert-Skala darzustellen. Die Entfernung eines jeden Punktes zur Grundlinie des Hautabdruckes kann durch die speziell entwickelte Bild-Digitalisierungstechnik und die Software des Skin Visiometers VS 400 in mm berechnet werden.

Die so gewonnenen Meßdaten wurden zentral nach eingehender Plausibilitätsprüfung und Qualitätssicherung in einen Rechner übernommen. Die Auswertung wurde mit Hilfe der Software "Statgraphics" für Windows, Version 3.0 - Manugistics, USA durchgeführt. Neben der deskriptiven Statistik wurde der "Wilcoxon matched pairs signed rank test" eingesetzt, wobei $p \leq 0{,}05$ als Hinweis auf einen statistischen Unterschied gilt.

Als Ergebnis der zuvor angesprochenen Untersuchung ist festzuhalten, daß die Anwendung des Produktes 2LA nach 28 Tagen zu einer statistisch signifikanten Straffung der Haut gegenüber der Ausgangssituation und gegenüber der unbehandelten Situation führt. Weiterhin bewirkt die Anwendung der Zusammensetzung 2LA eine statistisch signifikante Reduktion der Faltentiefe gegenüber der Ausgangssituation, während sich die Hautrauhigkeit ebenfalls statistisch gesichert vermindert. Desweiteren zeigen die Testergebnisse deutlich, daß die Zusammensetzung 2LA der Zusammensetzung 2LB deutlich überlegen ist.

Diese zuvor wiedergegebenen Aussagen werden durch die Abbildungen 1 bis 3 quantifiziert.

**In-Vivo-Test von einer Formulierung in bezug auf seine Wirksamkeit zur Behandlung von Psoriasis und Neurodermitis**

Es wurde eine Mikroemulsion hergestellt, die die folgenden Inhaltsstoffe aufwies:

| INCI-Namen | Gew.% |
|---|---|
| Lecithin | 2,5 |
| Alcohol | 6 |
| Sweet Almond Oil | 10 |
| Avocado Oil | 5 |
| Calendula Oil | 5 |
| Tocopheryl Acetat | 0,1 |
| Glycerine | 10 |
| Aqua | 60,6 |
| Propylene Glycol | 0,5 |
| Phenoxyethanol | 0,1 |
| Sodium Carboxymethyl Betaglucan | 0,2 |
| Parfum | 0,01 |

Die zuvor wiedergegebenen Bezeichnungen der Inhaltsstoffe erfolgte nach der INCI-Declaration, wie diese dem zuvor zitierten Dictionary zu entnehmen ist.

Mit dieser, nachfolgend auch als Zusammensetzung D bezeichneten Formulierung wurde zunächst eine 31

Jahre alte Frau behandelt, wobei diese Patientin an Psoriasis guttata erkrankt ist. Hierbei waren der rechte Handrücken, der linke kleine Finger, beide Beine (Innen- und Außenseite mehrere ca. 1 cm große Stellen), das linke Bein (5 cm große Stelle oberhalb des Fußinnenknöchels, entstanden nach starker, zweigradiger Verbrennung) sowie der gesamte Oberkörper mit kleineren Herden befallen, wobei zusätzlich noch beide Ellenbogen stark verkrustet waren.

Zur Behandlung wurden die befallenen Herde mehrmals nacheinander eingecremt, bis ein Sättigungsgefühl der Haut erreicht wurde.

Bereits nach einer viertägigen Behandlung waren die auf dem rechten Handrücken befallenen Stellen mit Ausnahme einer ganz kleinen Stelle ausgeheilt. An beiden Beinen war ein deutlicher Rückgang der Hautrötung sowie des Befalls feststellbar. Der 5 cm große Herd zeigte ebenfalls eine deutliche Heilung und einen deutlichen Rückgang der Rötung.

Die Patientin berichtete über ein subjektiv empfundenes gutes Hautgefühl und über ihre geschmeidige Haut nach Anwendung der Zusammensetzung D.

Eine 43 Jahre alte Frau, die seit etwa 30 Jahren an Psoriasis erkrankt ist und die in den letzten 10 Jahren nur noch einzelne, sporadisch auftretende Herde der Erkrankung am Ellenbogen und der Kopfhaut aufwies, berichtete, daß sie durch Anwendung der Zusammensetzung D auf den sporadisch auftretenden Psoriasisbefall innerhalb von wenigen Tagen eine nahezu sofortige Heilung erzielte. Dieses positive Ergebnis veranlaßte die Patientin, die früher eingesetzte Cortisonsalbe nicht mehr anzuwenden, da die Zusammensetzung D bei einem plötzlich auftretenden Psoriasisbefall eine bessere Abhilfe brachte als die ursprünglich verwendete Cortisonsalbe.

Ein 33 Jahre alter Mann, der über die Stirn verteilte, ca. 1 cm große entzündliche und schuppige Flecken, die sich bis in den Haaransatz und hinter die Ohren erstrecken, aufwies, verwendete etwa 2 Monaten die Zusammensetzung D. Hierbei erfolgte die Anwendung der Zusammensetzung D täglich nach dem Duschen. Bereits nach einwöchiger Anwendung der Zusammensetzung D konnte festgestellt werden, daß die entzündlichen, schuppigen Flecken nahezu spurlos verschwunden waren, wobei nach Abbruch der Behandlung mit der Zusammensetzung D nahezu unmittelbar erneut diese entzündlichen Herde auftraten. Desweiteren berichtete der Patient, daß nach Auftragen der Zusammensetzung D eine deutliche Linderung des Juckreizes zu bemerken war, wobei desweiteren die Anwendung der Zusammensetzung D dem Patienten ein deutliches positives Hautgefühl vermittelte.

Ein 17-jähriger Mann mit einer deutlich am Körper feststellbaren Neurodermitis wurde während 2,5 Wochen täglich zweimal mit der Zusammensetzung D behandelt. Nach Ablauf dieser Behandlungszeit war die Neurodermitis restlos verschwunden. Sobald jedoch die Behandlung mit der Zusammensetzung D beendet wurde, trat der Neurodermitis-Befall sofort wieder auf. Dies führte dazu, daß der Patient die entsprechende Behandlung fortführte, was wiederum innerhalb von kürzester Zeit eine deutliche Heilung bewirkte.

Eine für die Wundheilung verwendbare cremeartige Zusammensetzung weist insbesondere die nachfolgend wiedergegebenen Inhaltsstoffe auf:

| INCI-Namen | Gew.% |
|---|---|
| Aqua | 70 |
| Sodium Carboxymethyl Betaglucan | 0,1 |
| Panthenol | 2 |
| Propylene Glycol | 0,25 |
| Alcohol | 0,5 |
| Glycerine | 1 |
| Pentylenglycol | 1 |
| Methylchloroisothiazolinone | 0,1 |
| Allantoin | 0,1 |
| Disodium EDTA | 0,1 |
| Diisopropyl Adipate | 3 |
| Rentinyl Acetate | 0,5 |
| Lecithin | 0,2 |
| Caprylic/Capric Triglyceride | 9 |
| Steareth-2 | 3 |
| Steareth-21 | 2 |
| Tocopheryl Acetat | 2 |
| Farnesol | 1 |
| Stearic Acid/Palmitic Acid | 1,5 |
| Polyacrylamide | 2,5 |

(fortgesetzt)

| INCI-Namen | Gew.% |
|---|---|
| Parfum | 0,15 |

Die vorstehend verwendeten Bezeichnungen entsprechen der Declaration, wie sie dem "International Cosmetic Ingredient Dictionary", 1995, veröffentlicht von "The Cosmetic, Toiletry, and Fragrance Association", Washington, zu entnehmen sind.

## Patentansprüche

1. Verfahren zur Herstellung von polymeren Glucanäther-Derivaten, die neben Monomereneinheiten der Formel I auch solche Monomereneinheiten der allgemeinen Formel II aufweisen,

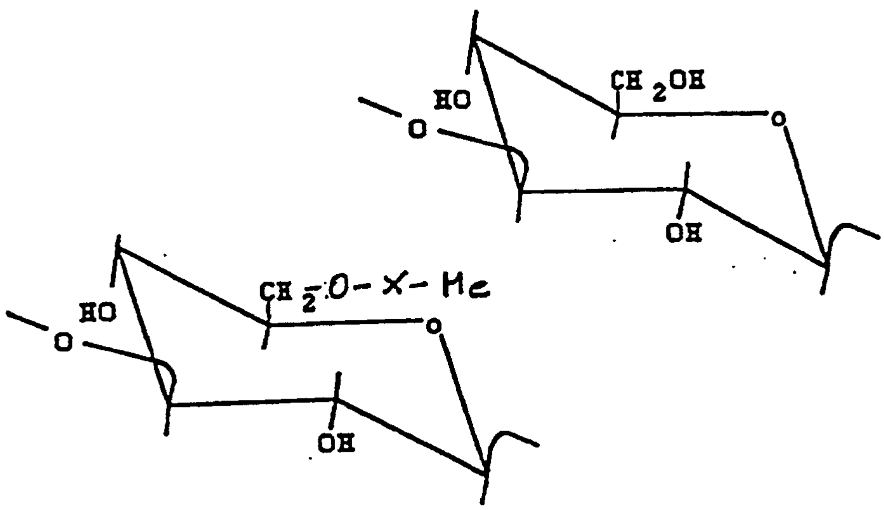

(Formel I)

(Formel II)

wobei die Monomereneinheiten der Formel I mit den Monomereneinheiten der Formel II über eine 1,3-beta-glykosidische Bindung verknüpft sind und in Formel II X eine $-CH_2-COO$-Gruppe, eine $-CH_2-CH_2-COO$-Gruppe, eine

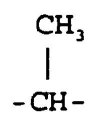

COO-Gruppe und/oder eine $-CH_2-CH_2-SO_3$-Gruppe und Me Wasserstoff, ein Alkali- und/oder ein Erdalkalimetall bedeuten, bei dem zur Herstellung der Glucanäther-Derivate

a) gereinigtes und weitestgehend lipidfreies Glucan in einem $C_1$-$C_4$-Alkohol unter Zusatz einer alkalischen Lösung behandelt wird,

b) hiernach ein Teil der alkalischen Lösung entfernt wird,

c) die dabei anfallende Suspension auf eine Temperatur zwischen 40 °C und 60 °C erwärmt und anschließend bei dieser Temperatur mit einer wäßrigen Lösung eines Salzes einer Halogencarbonsäure oder einer Halogensulfonsäure der nachfolgenden Formeln III

$$Y - CH_2-COO\ Me,$$
$$Y - CH_2-CH_2-COO\ Me,$$
$$Y - CH(CH_3)-COO\ Me \text{ und/oder}$$
$$Y - CH_2-CH_2-SO_3\ Me$$

(Formeln III)

umgesetzt wird, wobei in den Formeln III Y ein Halogen und Me ein Alkali- und/oder Erdalkalimetall bedeuten,

d) nach einer Reaktionszeit von einer Stunde bis vier Stunden das so gebildete Glucanäther-Derivat gewaschen und daß

e) das gewaschene Glucanäther-Derivat schonend getrocknet wird.

2. Verfahren nach Anspruch 1, **dadurch gekennzeichnet, daß** das gemäß Stufe a) gereinigte und weitestgehend lipidfreie Glucan in Isopropanol unter Zusatz einer alkalischen Lösung behandelt wird.

3. Verfahren nach Anspruch 1 oder 2, **dadurch gekennzeichnet, daß** das gereinigte und weitestgehend lipidfreie Glucan bei Raumtemperatur unter Zusatz der alkalischen Lösung behandelt wird.

4. Verfahren nach Anspruch 3, **dadurch gekennzeichnet, daß** das gereinigte und weitestgehend lipidfreie Glucan zwischen 10 Stunden und 25 Stunden unter Zusatz der alkalischen Lösung behandelt wird.

5. Verfahren nach Anspruch 4, **dadurch gekennzeichnet, daß** das gereinigte und weitestgehend lipidfreie Glucan zwischen 12 Stunden und 20 Stunden unter Zusatz der alkalischen Lösung behandelt wird.

6. Verfahren nach einem der vorangehenden Ansprüche, **dadurch gekennzeichnet, daß** das gereinigte und weitestgehend lipidfreie Glucan in dem $C_1$-$C_4$-Alkohol in einem Massenverhältnis 1:10 bis 1:30 suspendiert wird, und daß die Suspension mit einer wäßrigen Natronlauge als alkalische Lösung versetzt wird.

7. Verfahren nach Anspruch 6, **dadurch gekennzeichnet, daß** das gereinigte und weitestgehend lipidfreie Glucan in dem $C_1$-$C_4$-Alkohol in einem Massenverhältnis 1:15 bis 1:25 suspendiert wird, und daß die Suspension mit einer wäßrigen Natronlauge als alkalische Lösung versetzt wird.

8. Verfahren nach einem der vorangehenden Ansprüche, **dadurch gekennzeichnet, daß** als alkalische Lösung eine 20 Gew.%ige bis 35 Gew.%ige Natronlauge in einem Massenverhältnis von Glucan zu Natronlauge zwischen 1: 1,5 und 1:3 verwendet wird.

9. Verfahren nach einem der vorangehenden Ansprüche, **dadurch gekennzeichnet, daß** nach dem Entfernen des Teiles der alkalischen Lösung und vor dem Erwärmen der Suspension eine weitere Menge des $C_1$-$C_4$-Alkohols zugesetzt wird.

10. Verfahren nach Anspruch 9, **dadurch gekennzeichnet, daß** die zugesetzte Menge des $C_1$-$C_4$-Alkohols der Menge der zum Teil entfernten alkalischen Lösung entspricht.

11. Verfahren nach einem der vorangehenden Ansprüche, **dadurch gekennzeichnet, daß** das gebildete Glucanäther-Derivat nach Ablauf der Reaktionszeit durch Zusatz einer $C_1$-$C_4$-Alkohol/Wasser-Mischung gewaschen wird.

12. Verfahren nach einem der vorangehenden Ansprüche, **dadurch gekennzeichnet, daß** das Reaktionsgemisch nach Ablauf der Reaktionszeit durch Zusatz einer Säure, vorzugsweise Salzsäure, auf einen pH-Wert zwischen 6,8 und 7,5 neutralisiert wird.

13. Verfahren nach Anspruch 11 oder 12, **dadurch gekennzeichnet, daß** das gewaschene und/oder neutralisierte

Glucanäther-Derivat durch Zusatz des $C_1$-$C_4$-Alkohols aus der wäßrigen Lösung ausgefällt wird.

**14.** Verfahren nach einem der vorangehenden Ansprüche, **dadurch gekennzeichnet, daß** das Glucanäther-Derivat im Vakuum bei 40 °C bis 80 °C getrocknet wird.

## Claims

**1.** A method for the manufacturing of polymer glucan ether derivatives, which contain in addition to monomer units of the formula I also such monomer units of the general formula II,

(formula I)

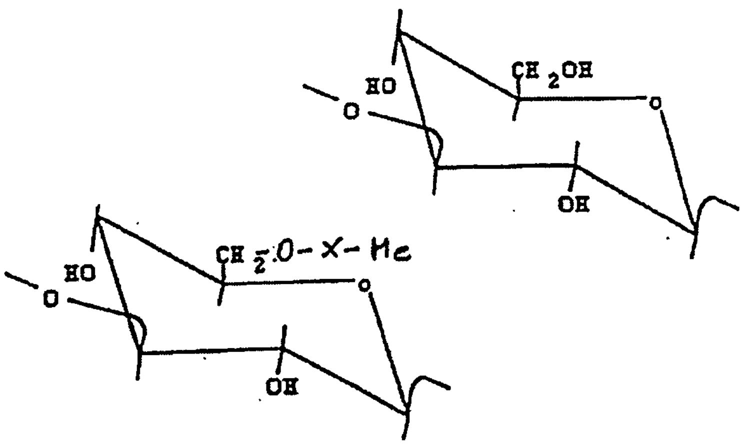

(formula II)

whereby the monomer units of the formula I are connected with the monomer units of the formula II by a 1,3-beta-glycosidic bond and whereby in formula II X means a $-CH_2-COO$-group, a $-CH_2-CH_2-COO$-group, a

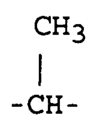

COO-group and/or a $-CH_2-CH_2-SO_3$-group and Me means hydrogen, an alkali- and/or an alkaline-earth metal, according to which

a) a purified and mainly lipid-free glucan is treated in a $C_1$-$C_4$-alcohol by adding an alkaline solution,

b) hereafter a part of the alkaline solution is removed,

c) the emerging suspension is heated to a temperature of between 40 °C and 60 °C and subsequently reacted at this temperature with an aqueous solution of a salt of a haloacid or a halogen sulpho acid of the following formula III

$Y - CH_2-COO\ Me,$

$Y - CH_2-CH_2-COO\ Me,$

$$\begin{array}{l} \quad CH_3 \\ \quad | \\ Y - CH\text{-}COO\ Me \text{ and/or} \\ Y - CH_2\text{-}CH_2\text{-}SO_3\ Me, \end{array} \qquad \text{(formula III)}$$

whereby in the formula III Y means a halogen and Me means an alkali- and/or alkaline-earth metal,

d) after a reaction time of one hour to four hours the thus formed glucan ether derivative is washed and

e) the washed glucan ether derivative is gently dried.

2. The method according to claim 1, **characterised in that** the purified and mainly lipid-free glucan according to step a) is treated in isopropanol by adding an alkaline solution.

3. The method according to claim 1 or 2, **characterised in that** the purified and mainly lipid-free glucan is treated at room temperature by adding the alkaline solution.

4. The method according to claim 3, **characterised in that** the purified and mainly lipid-free glucan is treated between 10 hours to 25 hours by adding the alkaline solution.

5. The method according to claim 4, **characterised in that** the purified and mainly lipid-free glucan is treated between 12 hours to 20 hours by adding the alkaline solution.

6. The method according to one of the preceding claims, **characterised in that** the purified and mainly lipid-free glucan is suspended in the $C_1$-$C_4$-alcohol in a mass ratio of 1:10 to 1:30 and that as alkaline solution an aqueous sodium hydroxide solution is added to the suspension.

7. The method according to claim 6, **characterised in that** the purified and mainly lipid-free glucan is suspended in $C_1$-$C_4$-alcohol in a mass ratio of 1:15 to 1:25 and that as alkaline solution an aqueous sodium hydroxide solution is added to the suspension.

8. The method according to claim 7, **characterised in that** as alkaline solution a sodium hydroxide solution of 20 % by weight to 35 % by weight is used in a mass ratio of glucan to the sodium hydroxide solution of between 1:1,5 and 1:3.

9. The method according to one of the preceding claims, **characterised in that**, after removing the part of the alkaline solution and before heating the suspension, a further amount of the $C_1$-$C_4$-alcohol is added.

10. The method according to claim 9, **characterised in that** the added amount of the $C_1$-$C_4$-alcohol corresponds to the amount of the partly removed alkaline solution.

11. The method according to one of the preceding claims, **characterised in that**, after the expiration of the reaction time, the formed glucan ether derivative is washed by adding a $C_1$-$C_4$-alcohol-/water-mixture.

12. The method according to one of the preceding claims, **characterised in that**, after the expiration of the reaction time, the reaction mixture is neutralised to an pH-value of between 6,8 and 7,5 by adding an acid, preferably hydrochloric acid.

13. The method according to claim 11 or 12, **characterised in that** the washed and/or neutralised glucan ether derivative is precipitated from the aqueous solution by adding the $C_1$-$C_4$-alcohol.

14. The method according to one of the preceding claims, **characterised in that** the glucan ether derivative is dried

in a vacuum at 40 °C to 80 °C.

**Revendications**

**1.** Procédé de préparation de dérivés d'éthers de glucane polymères qui, outre des unités monomères de formule I, présentent aussi des unités monomères de la formule générale II,

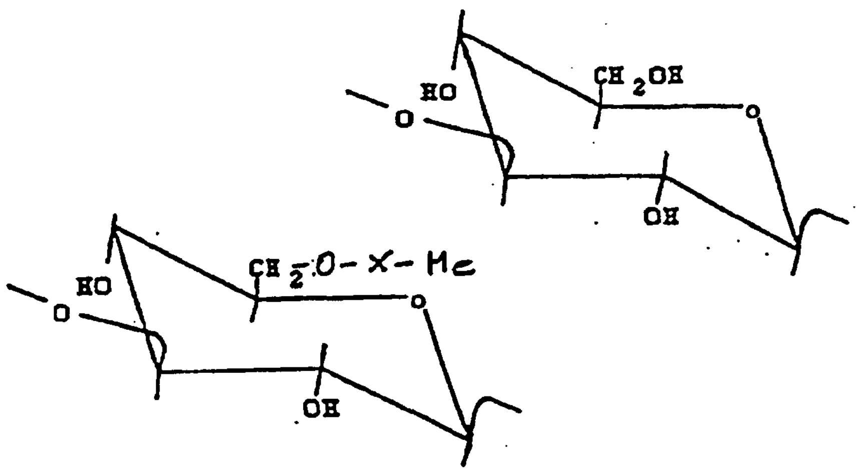

(formule I)

(formule II)

où les unités monomères de formule I sont liées aux unités monomères de formule II via une liaison 1,3-β-glycosidique et, dans la formule II, X représente un groupe $-CH_2-COO-$, un groupe $-CH_2CH_2-COO-$, un groupe - CH-COO et/ou un groupe $-CH_2-CH_2-SO_3$ et

|
$CH_3$

Me représente de l'hydrogène ou un métal alcalin et/ou alcalino-terreux, dans lequel, pour la préparation des dérivés d'éthers de glucane,

a) on traite du glucane purifié et pratiquement exempt de lipides dans un alcool en $C_1$ à $C_4$ avec addition d'une solution alcaline,
b) on élimine ensuite une partie de la solution alcaline,
c) on chauffe la suspension ainsi obtenue à une température comprise entre 40°C et 60°C et ensuite, à cette température, on la fait réagir avec une solution aqueuse d'un sel d'un acide carboxylique halogéné ou d'un acide sulfonique halogéné des formules III ci-après

$Y - CH_2 - COO\ Me,$

$Y - CH_2 - CH_2 - COO\ Me$ (formules III)

$CH_3$
|
$Y - CH - COO\ Me$, et/ou

$Y - CH_2 - CH_2 - SO_3\ Me$

et dans les formules III Y représente un halogène et Me un métal alcalin et/ou un métal alcalino-terreux,
d) après un temps de réaction de une heure à quatre heures, on lave le dérivé d'éther de glucane ainsi obtenu et
e) on sèche avec précaution le dérivé d'éther de glucane lavé.

**2.** Procédé selon la revendication 1, **caractérisé en ce que** le glucane purifié et pratiquement exempt de lipides selon l'étape a) est traité dans de l'isopropanol avec addition d'une solution alcaline.

**3.** Procédé selon la revendication 1 ou 2, **caractérisé en ce que** le glucane purifié et pratiquement exempt de lipides est traité à température ambiante avec addition de la solution alcaline.

**4.** Procédé selon la revendication 3, **caractérisé en ce que** le glucane purifié et pratiquement exempt de lipides est traité pendant 10 heures à 25 heures avec addition de la solution alcaline.

**5.** Procédé selon la revendication 4, **caractérisé en ce que** le glucane purifié et pratiquement exempt de lipides est traité pendant 12 heures à 20 heures avec addition de la solution alcaline.

**6.** Procédé selon l'une quelconque des revendications qui précèdent, **caractérisé en ce que** le glucane purifié et pratiquement exempt de lipides est mis en suspension dans l'alcool en $C_1$ à $C_4$ en un rapport pondéral de 1 : 10 à 1 : 30, et que la suspension est mélangée à de la soude caustique aqueuse en tant que solution alcaline.

**7.** Procédé selon la revendication 6, **caractérisé en ce que** le glucane purifié et pratiquement exempt de lipides est mis en suspension dans l'alcool en $C_1$ à $C_4$ en un rapport pondéral de 1 : 15 à 1 : 25, et que la suspension est mélangée à de la soude caustique aqueuse en tant que solution alcaline.

**8.** Procédé selon l'une quelconque des revendications qui précèdent, **caractérisé en ce que** l'on utilise comme solution alcaline de la soude caustique d'une concentration de 20% en poids à 35% en poids, en un rapport pondéral du glucane à la soude caustique compris entre 1 : 1,5 et 1 : 3.

**9.** Procédé selon l'une quelconque des revendications qui précèdent, **caractérisé en ce qu'**après l'élimination de la partie de la solution alcaline et avant le chauffage de la suspension, on ajoute un complément de l'alcool en $C_1$ à $C_4$.

**10.** Procédé selon la revendication 9, **caractérisé en ce que** la quantité ajoutée de l'alcool en $C_1$ à $C_4$ correspond à la quantité de solution alcaline partiellement éliminée.

**11.** Procédé selon l'une quelconque des revendications qui précèdent, **caractérisé en ce que** le dérivé d'éther de glucane formé au terme du temps de réaction est lavé par addition d'un mélange d'alcool en $C_1$ à $C_4$ et d'eau.

**12.** Procédé selon l'une quelconque des revendications qui précèdent, **caractérisé en ce que** le mélange réactionnel, au terme du temps de réaction, est neutralisé par addition d'un acide, de préférence de l'acide chlorhydrique, jusqu'à un pH compris entre 6,8 et 7.

**13.** Procédé selon la revendication 11 ou 12, **caractérisé en ce que** le dérivé d'éther de glucane lavé et/ou neutralisé est précipité de la solution aqueuse par addition de l'alcool en $C_1$ à $C_4$.

**14.** Procédé selon l'une quelconque des revendications qui précèdent, **caractérisé en ce que** le dérivé d'éther de glucane est séché sous vide à une température de 40°C à 80°C.

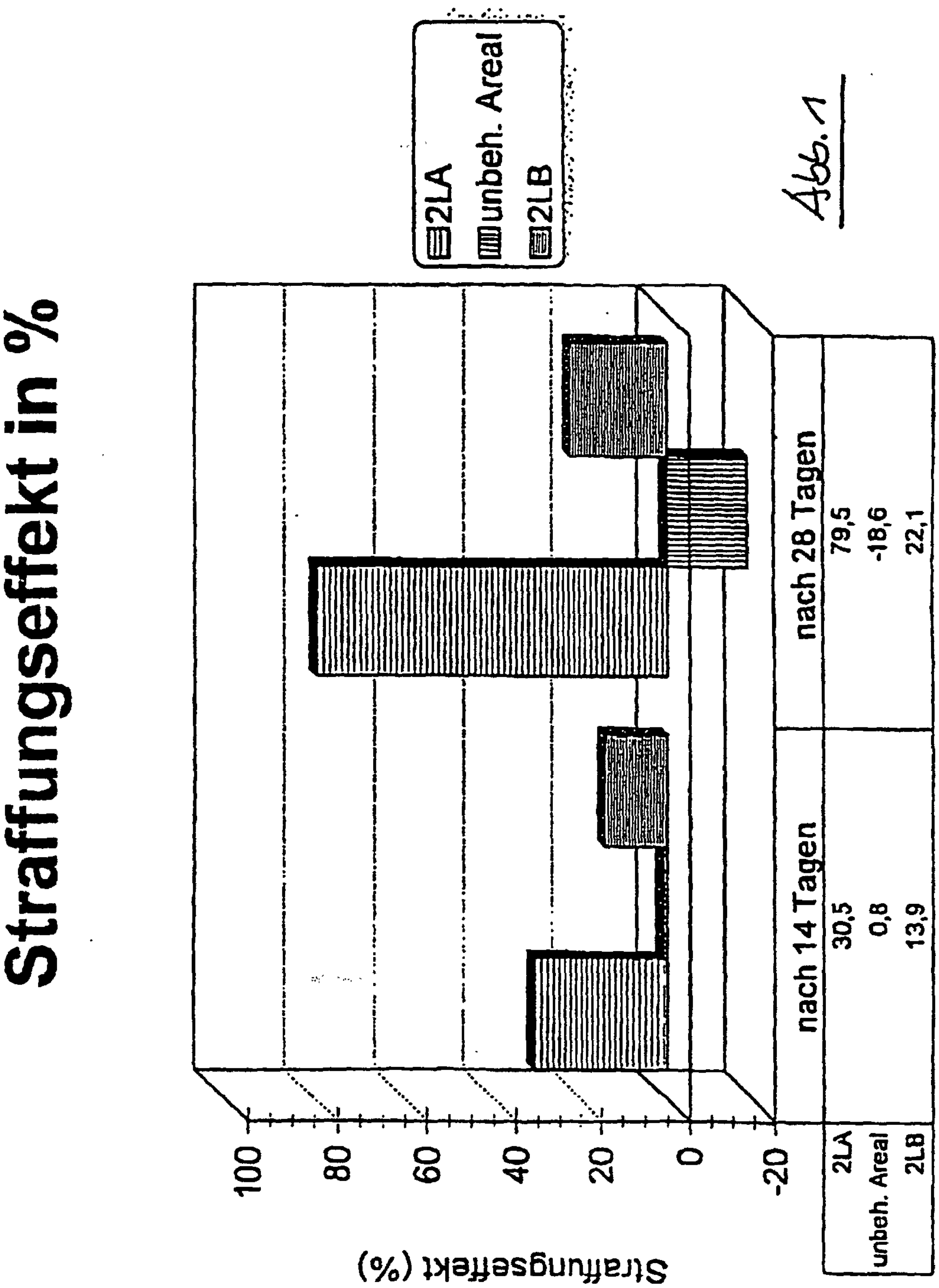
Straffungseffekt in %
Straffungseffekt (%)
100
80
60
40
20
0
-20
2LA
unbeh. Areal
2LB
nach 14 Tagen
30,5
0,8
13,9
nach 28 Tagen
79,5
-18,6
22,1

Abb. 1

**Reduktion der Faltentiefe in % bezogen auf den Ausgangswert**

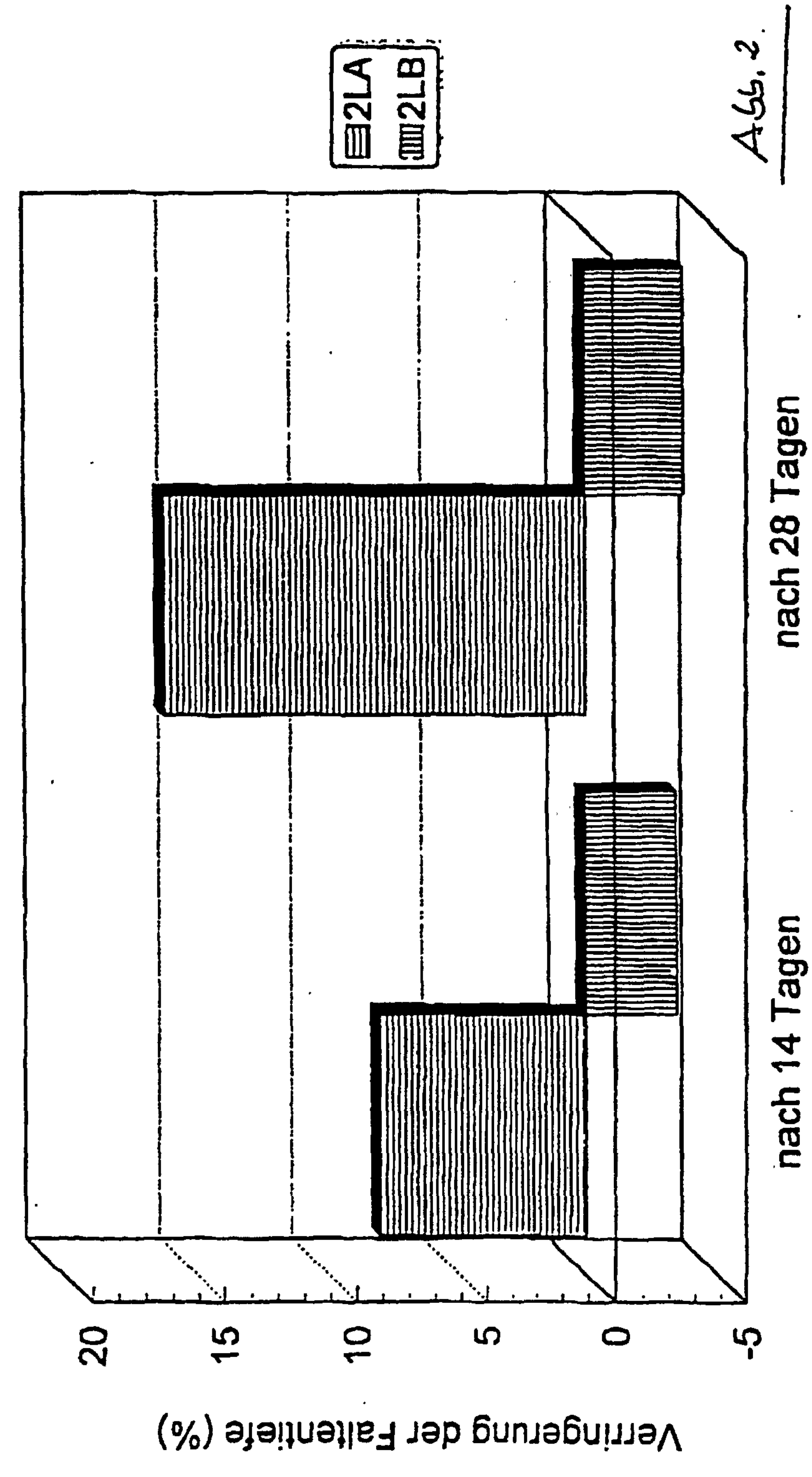


Abb. 2

Glättungseffekt in % bezogen auf den Ausgangswert

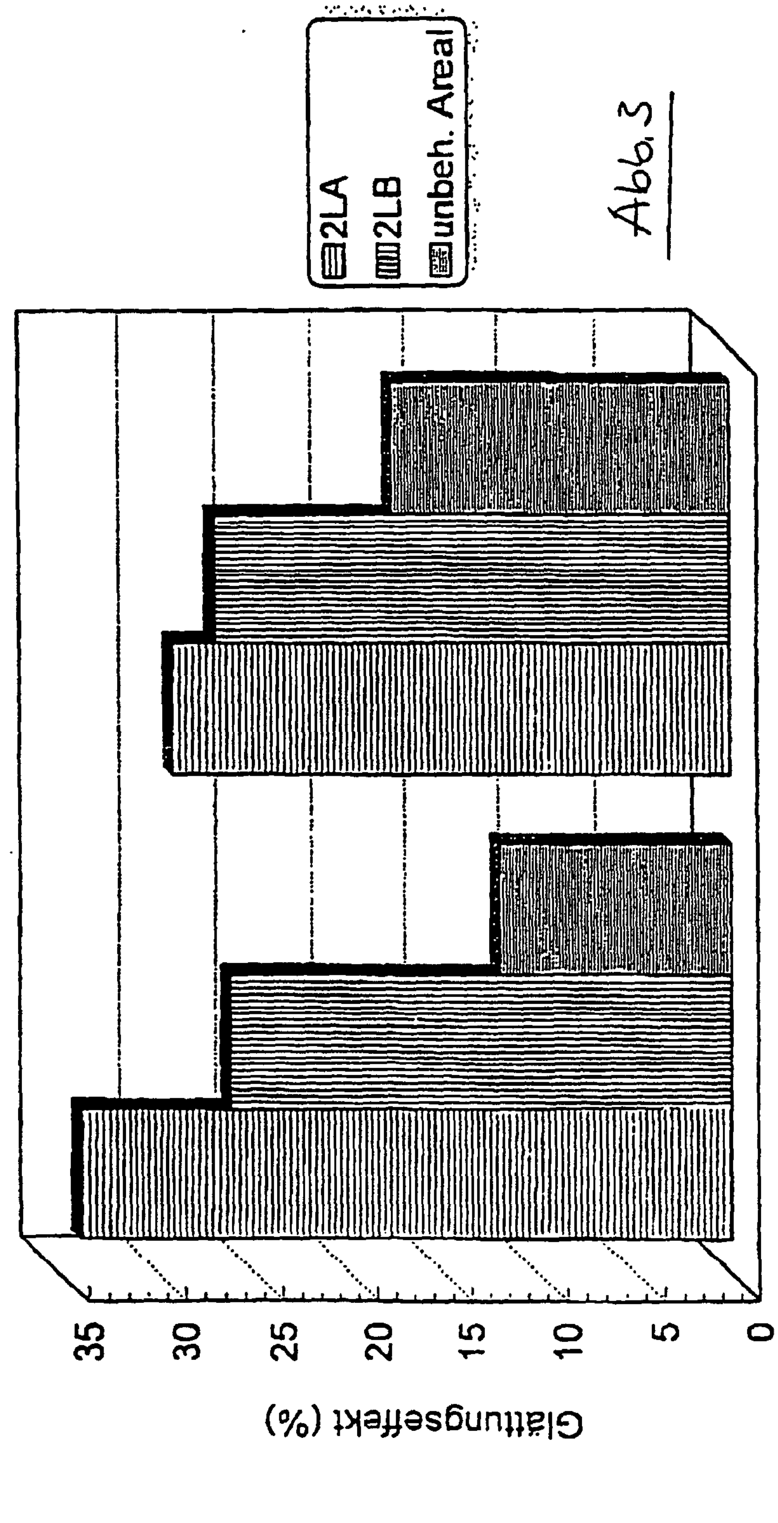


Abb. 3